Europäisches Patentamt

(19) European Patent Office

Office européen des brevets

(11) EP 0 931 769 A1

## (12) DEMANDE DE BREVET EUROPEEN

(43) Date de publication:
28.07.1999 Bulletin 1999/30

(51) Int. Cl.$^6$: C02F 5/12, C02F 1/50

(21) Numéro de dépôt: 98440011.9

(22) Date de dépôt: 27.01.1998

(84) Etats contractants désignés:
AT BE CH DE DK ES FI FR GB GR IE IT LI LU MC
NL PT SE
Etats d'extension désignés:
AL LT LV MK RO SI

(71) Demandeur:
HEINRICH ENVIRONNEMENT
Entreprise unipersonnelle à responsabilité
limitée
67120 Molsheim (FR)

(72) Inventeur: Behr, François
67120 - Molsheim (FR)

(74) Mandataire: Lepage, Jean-Pierre
c/o Innovations et Prestations S.A.,
4, rue de Haguenau
67000 Strasbourg (FR)

### (54) Nettoyant d'installations et procédé de nettoyage et de désinfection de telles installations

(57)     La présente invention est un nettoyant d'installations telles que des installations de stockage ou de distribution d'eau. Il est caractérisée par le fait qu'il comprend du chlorure de benzalkonium permettant d'éliminer les dépôts et incrustations organiques se formant au sein des installations, sans nuire à l'environnement.

L'invention concerne également un procédé de nettoyage et de désinfection de ces installations. Il est caractérisé en ce qu'on applique en deux phases successives sur les parois et les éléments à traiter dans les installations :

-     le nettoyant selon l'invention;
-     une solution désinfectante pour l'élimination de toute présence bactérienne et fongique résiduelle.

Application dans le domaine de la distribution d'eau.

EP 0 931 769 A1

Printed by Xerox (UK) Business Services
2.16.7/3.6

## Description

La présente invention est un nettoyant d'installations telles que des installations de stockage ou de distribution d'eau.

Elle concerne également un procédé de nettoyage et de désinfection de ces installations.

La propreté de telles installations est indispensable afin d'éviter des problèmes pathologiques chez les utilisateurs tels que des affections intestinales ou encore des épidémies de types variés telles que des hépatites. En effet, les installations d'eau sont destinées à délivrer de l'eau à différents utilisateurs et essentiellement de l'eau potable. La propreté de l'eau est donc indispensable.

L'invention pourra également trouver son application dans d'autres domaines que celui de la distribution d'eau, par exemple dans des réseaux ou des réserves d'autres liquides.

Plus spécifiquement dans le domaine des réservoirs ou des réseaux d'eau potable, des dépôts et incrustations apparaissent après un certain temps de fonctionnement sur les parois des installations.

Ces dépôts et incrustations peuvent être de différents types et se présentent sous différents aspects tels qu'un aspect floconneux ou encore granuleux, spongieux ou colloïdal.

Les dépôts et incrustations sont parfois d'origine chimique et sont fonction de la composition de l'eau. On observe alors la formation de dépôts minéraux tels que des carbonates ou des sulfates de calcium avec le plus souvent des charges métalliques telles que des oxydes de fer, du manganèse ou d'autres métaux. Des sulfures sont également observés par la réduction des sulfates.

D'autres dépôts et incrustations sont d'origine biologique. Ils sont formés par des algues, des champignons ou encore des bactéries. Ces sédiments biologiques parviennent à se développer de par la présence dans les eaux mêmes épurées de produits azotés ainsi que par l'existence de nitrate ou de nitrite.

On observe souvent une prolifération des deux types de dépôts et incrustations précités étant donné leur interaction. Par exemple, le métabolisme des micro-organismes entraîne des réactions chimiques favorisant la prolifération d'incrustations comme des oxydes ou des sulfures de fer.

Le développement au cours de l'utilisation de l'installation de ces dépôts et incrustations est particulièrement nuisible surtout si les installations ont pour objet la délivrance d'eau potable. Pour remédier à ce problème, on peut tout d'abord procéder à un nettoyage de type mécanique des installations. Il s'agit alors de parvenir à détacher des parois des installations les dépôts et incrustations. Ce nettoyage mécanique est fastidieux, difficile car les incrustations sont particulièrement adhérentes. L'intervention humaine est par ailleurs longue dans les installations difficiles d'accès.

Afin de trouver un système de nettoyage plus efficace, on a développé des procédés chimiques. On utilise alors une composition chimique unique appliquée sur les parois des installations.

C'est ainsi que l'on connaît actuellement des compositions chimiques comportant des éléments de nettoyage et des éléments de désinfection des installations.

Ces éléments sont le plus souvent des acides mélangées à des oxydants sous forme de chlore et de permanganate de potassium.

Les compositions actuellement utilisées ne sont pas sans danger et sans inconvénient notamment pour l'environnement.

En effet, le chlore est délicat à mettre en oeuvre étant donné son instabilité de stockage et les problèmes de rejet qu'il pose. Les éléments chlorés peuvent provoquer des précipitations de calcaire dans une eau dure ce qui les rend également parfois inefficace.

En ce qui concerne le permanganate de potassium, celui-ci engendre des dépôts de dioxyde de manganèse responsables d'une corrosion importante des installations. Le permanganate de potassium a également un effet nocif sur l'environnement de par sa toxicité.

Par ailleurs, les compositions actuelles incluent des éléments de nettoyage et des éléments de désinfection dans un seul produit. Les compositions chimiques ont alors l'inconvénient de ne permettre qu'un choix restreint de composés car ceux-ci doivent être susceptibles de se tolérer mutuellement.

On limite ainsi le choix des éléments de nettoyage et des éléments de désinfection ce qui est dommageable sur le plan de l'efficacité des produits et également sur le plan de la protection de l'environnement puisqu'il n'est pas toujours possible de choisir des éléments biodégradables ou facilement tolérables par l'écosystème.

Par ailleurs, l'efficacité antifongique et bactéricide des compositions actuelles est relativement faible par l'utilisation de compositions d'acide et d'oxydants chlorés ou de permanganate de potassium. Il existe donc un risque de mauvaise désinfection des installations. Ce risque est très dangereux puisqu'il est susceptible d'engendrer des épidémies ou des développements d'affections alors que les installations sont censées être propres.

Un autre inconvénient des compositions actuelles est que leur odeur est particulièrement désagréable. Les personnes chargées de la mise en oeuvre de ces compositions dans les installations d'eau sont donc amenées à effectuer un travail pénible pour lequel elles doivent le plus souvent porter des masques.

De plus, les compositions actuelles sont le plus souvent incolores ce qui ne permet pas de les distinguer entre elles et vis-à-vis d'autres solutions. Cela peut être particulièrement dangereux notamment si l'on confond une composition diluée avec une composition très concentrée.

La présente invention est un nettoyant d'installations et un procédé de nettoyage et de désinfection qui pallient les inconvénients des compositions actuelles. En premier lieu, elle offre un nettoyage efficace des installations sans nuire à l'environnement.

Pour ce faire, l'invention a l'avantage de comprendre des éléments facilement tolérés par l'environnement.

Par ailleurs, le présent nettoyant a l'avantage de cibler spécifiquement le nettoyage des installations et de permettre son utilisation de façon conjuguée avec une solution désinfectante. De cette façon, l'invention permet l'utilisation de composés remplissant à la fois les conditions d'efficacité dans le nettoyage et la désinfection et la tolérance vis-à-vis de l'environnement. Par ailleurs, les problèmes actuellement posés par la stabilité des compositions n'existent plus étant donné que la solution désinfectante est dissociée de la solution nettoyante.

Le nettoyant ici présenté a également l'avantage d'offrir une capacité de nettoyage aussi efficace sur le plan des dépôts et incrustations d'origine organique que ceux d'origine minérale.

Sur ce plan, l'utilisation d'un composé particulier à savoir de chlorure de benzalkonium est particulièrement intéressant puisqu'il permet à la fois l'homogénéité de la solution et la dissolution de toutes salissures d'origine organique.

Un autre but de l'invention est d'éviter toute dégradation des parois des installations par l'action corrosive des acides. Pour ce faire, le nettoyant selon l'invention présente un agent inhibiteur de corrosion qui évitera toute dégradation des parties métalliques des parois.

Un autre objet de l'invention est de proposer un nettoyant présentant une odeur et une couleur spécifiques. Sur le plan de l'odeur, l'invention a l'avantage de proposer une formulation dont l'odeur n'est pas désagréable ce qui est particulièrement important pour améliorer les conditions de travail. Par ailleurs, la présente invention aura une couleur spécifique permettant de ne pas la confondre avec un autre produit.

Tous les composants du nettoyant selon la présente invention ont une toxicité faible et sont sans danger pour la santé.

Le procédé selon l'invention a également l'avantage d'être pratique à mettre en oeuvre dans les installations en deux phases successives. L'utilisation de deux produits distincts en deux phases est très efficace et respecte mieux l'environnement.

D'autres buts et avantages apparaîtront au cours de la description qui va suivre qui n'est cependant donnée qu'à titre indicatif.

La présente invention concerne un nettoyant d'installations telles que des installations de stockage ou de distribution d'eau, caractérisé en ce qu'il comprend du chlorure de benzalkonium permettant d'éliminer les dépôts et incrustations organiques se formant au sein des installations, sans nuire à l'environnement.

L'invention concerne également un procédé de nettoyage et de désinfection d'installations telles que des installations de stockage ou de distribution d'eau, caractérisée en ce qu'on applique en deux phases successives sur les parois et les éléments à traiter dans les installations :

-    le nettoyant selon l'invention,
-    une solution désinfectante pour l'élimination de toute présence bactérienne et fongique résiduelle.

Le nettoyant d'installations selon l'invention comprend tout d'abord du chlorure de benzalkonium.

Le chlorure de benzalkonium assure non seulement la dissolution des salissures d'origine organique mais aussi l'homogénéité de la solution. En effet, le chlorure de benzalkonium remplit la fonction d'agent tensio-actif. Il peut se combiner avec de l'isopropanol afin de compléter son action.

Pour compléter l'action du chlorure de benzalkonium, on utilisera préférentiellement un ou plusieurs composés acides assurant l'élimination des dépôts et incrustations d'origine minérale.

Le ou les composants acides pourront notamment être de l'acide chlorhydrique, de l'acide phosphorique et de l'acide formique. Ces acides permettent en effet d'éliminer les salissures des parois des installations tout en évitant une trop forte dégradation des parois.

Les composants acides du nettoyant selon l'invention permettent d'éliminer de façon très efficace les dépôts et incrustations d'origine minérale. Ceci est particulièrement important étant donné que le calcaire et autres carbonates représentent une part prépondérante des dépôts.

La dégradation des dépôts se fait selon la réaction suivante qui est donnée à titre d'exemple pour un dépôt de carbonate de calcium et l'utilisation d'un acide chlorhydrique :

$$CaCO_3 + 2HCl \rightarrow CaCl_2 + H_2O + CO_2$$

De la même façon, les hydroxydes sont dissout en milieu acide selon la réaction suivante dans le cas particulier de l'utilisation d'acide chlorhydrique :

$$Ca(OH)_2 + 2HCL \rightarrow CaCl_2 + 2H_2O$$

Dans la pratique, on constate que les composants acides entraînent une faible dégradation des parois des installations. A titre indicatif, on a mis en évidence qu'il fallait plus d'une vingtaine de nettoyages successifs pour dégrader des parois en béton sur une épaisseur de un millimètre. Cette usure chimique est négligeable par rapport à l'abrasion mécanique qui était mise en oeuvre jusqu'alors.

On utilisera par ailleurs préférentiellement de l'hexaméthylène-tetramine. On a en effet remarqué son action très avantageuse d'inhibiteur de corrosion. L'incorporation d'un inhibiteur de corrosion et particulièrement de l'hexaméthylène-tetramine évite la dégradation des parties métalliques des installations.

Ainsi, par utilisation conjointe d'acides et de chlorure de benzalkonium, on parvient à élaborer une solution assurant la dégradation d'une part des salissures minérales et d'autre part des dépôts et incrustations organiques. Par ailleurs, ce mélange est facilement toléré par l'environnement ce qui n'était pas le cas des compositions utilisées jusqu'alors comportant de fortes quantités de chlore et de permanganate de potassium.

Essentiellement, c'est en solution que l'on utilisera le nettoyant selon l'invention. Plus précisément, l'eau chaude, par exemple à une température de l'ordre de 50 °C, donne de bons résultats.

Dans un autre mode de réalisation de l'invention, le nettoyant contient un composé lui conférant une odeur spécifique. On pourra utiliser pour ce faire un composé mentholé permettant de distinguer par l'odeur le nettoyant mais aussi de masquer les odeurs désagréables de certains composants du mélange. L'utilisation d'un tel composé est particulièrement intéressante puisque jusqu'ici il fallait utiliser des masques pour supporter les odeurs désagréables des compositions existantes.

Par ailleurs, le composé mentholé permet de conférer une odeur spécifique à la composition et donc de permettre de la distinguer aisément d'une autre solution afin d'éviter tout risque d'erreur dans la manipulation des produits.

Dans un autre mode de réalisation, le nettoyant contient un colorant permettant de lui conférer une couleur spécifique. Ce colorant pourra être du chlorure de cuivre qui, en doses très faibles, ne présente aucun risque pour l'environnement. Là encore, le colorant assure une différenciation particulièrement avantageuse de la solution par rapport aux autres produits susceptibles d'être utilisés.

Afin d'optimiser l'action de nettoyage de la présente invention, elle pourra contenir un séquestrant d'ions métalliques sous forme d'acide citrique. Ce composé est efficace et inoffensif.

En terme de proportions pondérales, on pourra utiliser 30 à 60 % d'acide et 0.05 à 0.5 % de Chlorure de benzalkonium. On donne ci-après un exemple de formulation du nettoyant selon l'invention. Les proportions sont données à titre indicatif mais ne limitent en rien l'invention à cette répartition pondérale.

| COMPOSANT | Proportion pondérale en % |
|---|---|
| Acide formique | 2 |
| Acide phosphorique | 9 |
| Acide chlorhydrique | 42.5 |
| Acide citrique | 4 |
| Chlorure de benzalkonium | 0.2 |
| Hexaméthylène-tetramine | 0.4 |
| Isopropanol | 4 |
| Chlorure de cuivre | 0.1 |
| Menthol | 0.03 |
| Eau | 37.8 |

On pourra également mettre en oeuvre un dosage particulièrement adapté à une utilisation d'entretien pour des installations faisant l'objet de nettoyages réguliers. On donne ci-après un exemple de formulation dans ce cadre d'application.

| COMPOSANT | Proportion pondérale en % |
|---|---|
| Acide formique | 3 |
| Acide phosphorique | 3.5 |
| Acide chlorhydrique | 25.5 |
| Acide citrique | 3.5 |
| Chlorure de benzalkonium | 0.1 |
| Hexaméthylène-tetramine | 0.4 |
| Isopropanol | 4 |
| Chlorure de cuivre | 0.4 |
| Menthol | 0.03 |
| Eau | 59.57 |

La présente invention concerne également un procédé de nettoyage et de désinfection d'installations.

A l'heure actuelle, on utilise les compositions existantes en une seule application pour nettoyer les installations. Le procédé selon l'invention se distingue des techniques actuelles en réalisant un nettoyage et une désinfection en deux phases successives.

Cette distinction de deux phases permet de ne pas utiliser dans une même composition les éléments nettoyants et les éléments désinfectants. Le résultat du nettoyage de la désinfection est en conséquence optimisé.

Par ailleurs, l'utilisation de deux solutions distinctes permet un plus large choix dans leur composition puisque des contraintes liées à la stabilité et l'homogénéité de la formule sont supprimées.

Selon le procédé ici décrit, on applique dans les installations, en deux phases successives :

- le nettoyant décrit plus haut;
- une solution désinfectante.

La solution désinfectante utilisée permet l'élimination de tout élément bactérien et fongique résiduel après l'utilisation du nettoyant.

On utilisera préférentiellement une solution désinfectante comprenant du peroxyde d'hydrogène. Ce composé est à la fois efficace et respectueux de l'environnement. Par ailleurs, il n'est pas nécessaire d'utiliser conjointement des catalyseurs de type métallique car ces éléments sont déjà présents dans les installations par exemple sous forme d'ions métalliques de fer ou de manganèse. De cette façon, on ne charge pas la solution désinfectante en éléments polluants et on exploite par ailleurs les éléments métalliques présents naturellement dans les installations.

Pour optimiser encore l'efficacité de la solution désinfectante, on pourra utiliser de l'acide peracétique.

Les nombreux travaux du déposant ont permis de montrer que l'acide peracétique constituait un excellent activateur de réactions. La solution désinfectante comprend ainsi de l'oxygène actif organique et permet de dégrader de façon systématique les polluants organiques.

L'acide peracétique et le peroxyde d'hydrogène ont donc une action conjuguée importante et une solution de nettoyage combinant du peroxyde d'hydrogène et de l'acide peracétique est d'une grande efficacité ce qui évite d'avoir recours à l'utilisation de chlore ou encore de permanganate de potassium qui sont plus nocifs pour l'environnement.

On donne dans le tableau suivant un exemple de formulation en proportions pondérales.

| COMPOSANT | Proportion pondérale en % |
|---|---|
| Peroxyde d'hydrogène | 70 à 85 |
| Acide peracétique | 15 à 30 |

On a par ailleurs constaté que l'eau oxygénée permettait l'oxydation des produits soufrés tels que des sulfures très fréquemment présents dans les réseaux de distribution d'eau et mal odorants.

Ainsi, contrairement à ce que laisse préjuger l'état actuel de la technique, l'invention parvient à un nettoyage et une désinfection particulièrement efficaces et larges tout en écartant l'utilisation de chlore, de permanganate de potassium et d'autres éléments nocifs pour l'environnement.

L'invention ici décrite est mise en oeuvre de façon très pratique.

Dans un premier temps, on applique le nettoyant sur les parois et autres éléments des installations. Sous l'effet des composants acides, les installations sont nettoyées de tous dépôts et incrustations d'origine minérale. Ceci s'effectue par ailleurs sans gêne pour la structure des installations puisque la dégradation des bétons est très limitée et que la corrosion des éléments métalliques des installations est évitée par l'utilisation d'un inhibiteur de corrosion tel que l'hexaméthylène-tetramine.

Le chlorure de benzalkonium assure l'élimination des dépôts et incrustations d'origine organique.

Pour l'utilisateur, la solution de nettoyage est d'utilisation très pratique puisqu'une couleur et une odeur spécifiques lui évitent tout risque d'erreur avec un autre produit et permet des conditions de travail améliorées de par l'odeur non désagréable de la solution.

L'efficacité du nettoyant selon l'invention est telle qu'une application longue n'est pas nécessaire ce qui évite de stopper trop longtemps les installations. La gêne pour le consommateur d'eau constituée par l'arrêt de la distribution est donc très réduite.

Consécutivement à l'action de la solution de nettoyage, on applique alors la solution désinfectante.

Celle-ci, constituée de peroxyde d'hydrogène et d'acide peracétique a une efficacité très importante remplaçant très avantageusement le chlore et le permanganate de potassium.

Toute présence bactérienne et fongique résiduelle est alors supprimée.

**Revendications**

1. Nettoyant d'installations telles que des installations de stockage ou de distribution d'eau, caractérisé en ce qu'il comprend du chlorure de benzalkonium permettant d'éliminer les dépôts et incrustations organiques se formant au sein des installations, sans nuire à l'environnement.

2. Nettoyant d'installations telles que des installations de stockage ou de distribution d'eau, selon la revendication 1, caractérisé en ce qu'il comprend un mélange de chlorure de benzalkonium et d'un ou plusieurs composants acides.

3. Nettoyant d'installations telles que des installations de stockage ou de distribution d'eau, selon la revendication 2, caractérisé en ce qu'il comprend 30 à 60 % d'acide et 0.05 à 0.5 % de chlorure de benzalkonium.

4. Nettoyant d'installations telles que des installations de stockage ou de distribution d'eau, selon l'une des revendications 1 à 3, caractérisé en ce qu'il comprend un agent inhibiteur de corrosion afin d'éviter la dégradation des parties métalliques des installations sous l'action du ou des composants acides.

5. Nettoyant d'installations telles que des installations de stockage ou de distribution d'eau, selon la revendication 4, caractérisée en ce que l'agent inhibiteur de corrosion est l'hexaméthylène-tetramine.

6. Nettoyant d'installations telles que des installations de stockage ou de distribution d'eau, selon l'une des revendications 1 à 5, caractérisée en ce qu'elle contient un colorant tel que du chlorure de cuivre pour lui conférer une couleur spécifique et un composé mentholé pour lui conférer une odeur spécifique et non désagréable.

7. Nettoyant d'installations telles que des installations de stockage ou de distribution d'eau, selon l'une des revendications 1 à 6, caractérisée en ce qu'elle est contient, en proportions pondérales, les composants suivants :

   - acide formique : 2 à 3 % ;
   - acide phosphorique : 3.5 à 9 % ;
   - acide chlorhydrique : 25.5 à 42.5 % ;
   - acide citrique : 3 à 4 % ;
   - chlorure de benzalkonium : 0.1 à 0.2% ;
   - isopropanol : 4 % ;
   - eau : 35 à 60 %.

8. Procédé de nettoyage et de désinfection d'installations telles que des installations de stockage ou de distribution d'eau, caractérisée en ce qu'on applique en deux phases successives sur les parois et les éléments à traiter dans les installations :

   - le nettoyant selon l'une des revendications 1 à 7;
   - une solution désinfectante pour l'élimination de toute présence bactérienne et fongique résiduelle.

9. Procédé de nettoyage et de désinfection d'installations, selon la revendication 8, caractérisé par le fait que la solution désinfectante utilisée comprend du peroxyde d'hydrogène.

10. Procédé de nettoyage et de désinfection d'installations, selon la revendication 9, caractérisé par le fait que la solution désinfectante comprend de l'acide péracétique optimisant l'action désinfectante du peroxyde d'hydrogène.

**Office européen des brevets**

## RAPPORT DE RECHERCHE EUROPEENNE

Numéro de la demande

EP 98 44 0011

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X | US 4 062 796 A (TOMMY R.GARDNER ET AL) 13 décembre 1977<br>* revendications 1-5 *<br>----- | 1,2 | C02F5/12<br>C02F1/50 |

|  |  |  | DOMAINES TECHNIQUES RECHERCHES (Int.Cl.6) |
|---|---|---|---|
|  |  |  | C02F |

Le présent rapport a été établi pour toutes les revendications

| Lieu de la recherche | Date d'achèvement de la recherche | Examinateur |
|---|---|---|
| LA HAYE | 23 juin 1998 | Fouquier, J-P |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
................................................................
& : membre de la même famille, document correspondant

EPO FORM 1503 03.82 (P04C02)